# NEW EUROPEAN PATENT SPECIFICATION

(11) **EP 3 023 095 B2**
(45) Date of publication and mention of the opposition decision: **08.11.2023**
(45) Mention of the grant of the patent: 16.05.2018
(21) Application number: 15199440.7
(22) Date of filing: 17.01.2005
(51) Int. Cl.: A61K 9/20

(54) **DIRECT COMPRESSION FORMULATION AND PROCESS**
DIREKTKOMPRIMIERTE FORMULIERUNG UND VERFAHREN
FORMULATION ET PROCEDE POUR COMPRESSION DIRECTE

(30) Priority: 20.01.2004 US 537706 P; 25.08.2004 US 604274 P
(43) Date of publication of application: 25.05.2016
(62) Divisional of application: 09157626.4
(73) Proprietor: Novartis AG, 4056 Basel (CH)
(72) Inventor: Kowalski, James, Belle Mead, NJ 08502 (US); Lakshman, Jay Parthiban, Bridgewater, NJ 08807 (US); Patel, Arun P., Succasunna, NJ 07876 (US)
(74) Representative: Carpmaels & Ransford LLP

(56) References cited:
- WO-A1-00/34241
- JP-A- 2003 327 532
- US-A1- 2003 078 247
- US-B1- 6 548 481
- EDWIN B VILLHAUER ET AL: "1-[[(3-Hydroxy-1-adamantyl)amino]acetyl]- 2-cyano-(S)-pyrrolidine: A Potent, Selective, and Orally Bioavailable Dipeptidyl Peptidase IV Inhibitor with Antihyperglycemic Properties", JOURNAL OF MEDICINAL CHEMISTRY, AMERICAN CHEMICAL SOCIETY, US, vol. 46, no. 13, 1 January 2003 (2003-01-01), pages 2774-2789, XP002663718, ISSN: 0022-2623, DOI: 10.1021/JM030091L [retrieved on 2003-05-24]
- LIEBERMANN H A ET AL: "Pharmaceutical Dosage Forms: Tablets", 1 January 1989 (1989-01-01), PHARMACEUTICAL DOSAGE FORMS-TABLETS/ ED. 2, XX, XX, PAGE(S) I-II, XP003027810, * page 197, paragraph 3 - page 198, paragraph 2 *

## Description

This invention relates to tablets especially tablets formed by direct compression of the dipeptidylpeptidase IV (DPP-IV) inhibitor compound, a process for the preparation thereof, to new pharmaceutical formulations , and new tableting powders comprising DPP-IV inhibitor formulations capable of being directly compressed into tablets, the DPP-IV inhibitor according to the invention is (S)-1-[(3-hydroxy-1-adamantyl)amino]acetyl-2-cyanopyrrolidine in free form or in acid addition salt form. The invention relates further to a process for preparing the tablets by blending the active ingredient and specific excipients into the new formulations and then directly compressing the formulations into the direct compression tablets.

DPP-IV inhibitors are known in the art. For example, DPP-IV inhibitors are in each case generically and specifically disclosed e.g. in WO 98/19998,DE19616 486 A1, WO 00/34241, WO 95/15309, WO 01/72290, WO 01/52825, WO 9310127, WO 9925719, WO 9938501, WO 9946272, WO 9967278 and WO 9967279.

DPP-IV inhibitors are described in the following patent applications; WO 02053548 especially compounds 1001 to 1293 and examples 1 to 124, WO 02067918 especially compounds 1000 to 1278 and 2001 to 2159, WO 02066627 especially the described examples, WO 02/068420 especially all the compounds specifically listed in the examples I to LXIII and the described corresponding analogues, even preferred compounds are 2(28), 2(88), 2(119), 2(136) described in the table reporting IC50, WO 02083128 especially examples 1 to 13, US 2003096846 especially the specifically described compounds, WO 2004/037181 especially examples 1 to 33 and compounds of claims 3 to 5, WO 0168603 especially compounds of examples 1 to 109, EP1258480 especially compounds of examples 1 to 60, WO 0181337 especially examples 1 to 118, WO 02083109 especially examples 1A to 1D, WO 030003250 especially compounds of examples 1 to 166, most preferably 1 to 8, WO 03035067 especially the compounds described in the examples, WO 03/035057 especially the compounds described in the examples, US2003216450 especially examples 1 to 450, WO 99/46272 especially compounds of claims 12, 14, 15 and 17, WO 0197808 especially compounds of claim 2, WO 03002553 especially compounds of examples 1 to 33, WO 01/34594 especially the compounds described in the examples 1 to 4, WO 02051836 especially examples 1 to 712, EP1245568 especially examples 1 to 7, EP1258476 especially examples 1 to 32, US 2003087950 especially the described examples, WO 02/076450 especially examples 1 to 128, WO 03000180 especially examples 1 to 162, WO 03000181 especially examples 1 to 66, WO 03004498 especially examples 1 to 33, WO 0302942 especially examples 1 to 68, US 6482844 especially the described examples, WO 0155105 especially the compounds listed in the examples 1 and 2, WO 0202560 especially examples 1 to 166, WO 03004496 especially examples 1 to 103, WO 03/024965 especially examples 1 to 54, WO 0303727 especially examples 1 to 209, WO 0368757 especially examples 1 to 88, WO 03074500 especially examples 1 to 72, examples 4.1 to 4.23, examples 5.1 to 5.10, examples 6.1 to 6.30, examples 7.1 to 7.23, examples 8.1 to 8.10, examples 9.1 to 9.30, WO 02038541 especially examples 1 to 53, WO 02062764 especially examples 1 to 293, preferably the compound of example 95 (2-{{3-(Aminomethyl)-4-butoxy-2-neopentyl-1-oxo-1,2 dihydro-6-isoquinolinyl}oxy}acetamide hydrochloride), WO 02308090 especially examples 1-1 to 1-109, examples 2-1 to 2-9, example 3, examples 4-1 to 4-19, examples 5-1 to 5-39, examples 6-1 to 6-4, examples 7-1 to 7-10, examples 8-1 to 8-8, examples 7-1 to 7-7 of page 90, examples 8-1 to 8-59 of pages 91 to 95, examples 9-1 to 9-33, examples 10-1 to 10-20, US 2003225102 especially compounds 1 to 115, compounds of examples 1 to 121, preferably compounds a) to z), aa) to az), ba) to bz), ca) to cz) and da) to dk), WO 0214271 especially examples 1 to 320 and US 2003096857, WO 2004/052850 especially the specifically described compounds such as examples 1 to 42 and compounds of claim 1, DE 102 56 264 A1 especially the described compounds such as examples 1 to 181 and the compounds of claim 5, WO 04/076433 especially the compounds specifically described, such as listed in table A, preferably the compounds listed in table B, preferably compounds I to XXXXVII, or compounds of claims 6 to 49, WO 04/071454 especially the specifically described compounds e.g. compounds 1 to 53 or compounds of tables la to If, or compounds of claims 2 to 55, WO 02/068420 especially the compounds specifically described, such as the compounds I to LXIII or Beispiele I and analogues 1 to 140 or Beispiele 2 and analogues 1 to 174 or Beispiele 3 and analogues 1, or Beispiele 4 to 5, or Beispiele 6 and analogues 1 to 5, or Beispiele 7 and analogues 1-3, or Beispiele 8 and analogue 1, or Beispiele 9, or Beispiele 10 and analogues 1 to 531 even preferred are compounds of claim 13, WO 03/000250 especially the compounds specifically described, such as the compounds 1 to 166, preferably compounds of examples 1 to 9, WO 03/024942 especially the compounds specifically described, such compounds 1 to 59, compounds of table 1 (1 to 68), compounds of claims 6, 7, 8, 9, WO 03024965 especially the compounds specifically described, such compounds 1 to 54, WO 03002593 especially the compounds specifically described, such compounds table 1 or of claims 2 to 15, WO 03037327 especially the compounds specifically described, such compounds of examples 1 to 209 WO 03/000250 especially the compounds specifically described, such as the compounds 1 to 166, preferably compounds of examples 1 to 9, WO 03/024942 especially the compounds specifically described, such compounds 1 to 59, compounds of table 1 (1 to 68), compounds of claims 6, 7, 8, 9, WO 03024965 especially the compounds specifically described, such compounds 1 to 54, WO 03002593 especially the compounds specifically described, such compounds table 1 or of claims 2 to 15, WO03037327 especially the compounds specifically described, such compounds of examples 1 to 209, WO 0238541, WO 0230890, U.S. application Serial No. 09/788,173 filed February 16, 2001 (attorney file LA50) especially the described examples, WO99/38501 especially the described examples, WO99/46272 especially the described examples and DE19616 486 A1 especially val-pyr, val-thiazolidide, isoleucyl-thiazolidide, isoleucyl-pyrrolidide, and fumar salts of isoleucyl-thiazolidide and isoleucyl-pyrrolidide, WO 0238541 especially the compounds specifically described, such compounds of examples 1 to 53, WO 03/002531 especially the compounds specifically described preferably the compounds listed on page 9 to 13, most preferably the compounds of examples 1 to 46 and even preferred compound of example 9, U.S. Patent No. 6,395,767 preferably compound of examples 1 to 109 most preferably compound of example 60.

Further preferred DPP-IV inhibitors include the specific examples disclosed in United States Patent Numbers 6124305 and US 6107317, International Patent Applications, Publication Numbers WO 9819998, WO 95153 09 and WO 9818763; such as 1[2-[(5 eyanopyridin-2-yl)aminoethylamino]acetyl-2-cyano-(S)-pyrrolidine and (2S)-I-[(2S)-2arnino-3,3-dimethylbutanoyl]-2-pyrrolidinecarbonitrile.

WO 9819998 discloses N-(N'-substituted glycyl)-2-cyano pyrrolidines, in particular 1-[2-[5-Cyanopyridin-2-yl] amino]- ethylamino] acetyl-2-cyano- (S)- pyrrolidine. Preferred compounds described in WO03/002553 are listed on pages 9 to 11 and are incorporated into the present application by reference. Published patent application WO 0034241 and published patent US 6110949 disclose N-substituted adamantyl-amino-acetyl-2-cyano pyrrolidines and N-(substituted glycyl)-4-cyano pyrrolidines respectively. DPP-IV inhibitors of interest are specially those cited in claims 1 to 4. In particular these applications describe the compound 1-[[(3-Hydroxy-1-adamantyl) amino]acetyl]-2-cyano-(S)-pyrrolidine (also known as LAF237).

WO 9515309 discloses amino acid 2- cyanopyrrolidine amides as inhibitors of DPP-IV and WO 9529691 discloses peptidyl derivates of diesters of alpha-aminoalkylphosphonic acids, particularly those with proline or related structures. DPP-IV inhibitors of interest are specially those cited in Table 1 to 8. In WO 01/72290 DPP-IV inhibitors of interest are specially those cited in example 1 and claims 1, 4,and 6. WO 9310127 discloses proline boronic esters useful as DPP-IV inhibitors. DPP-IV inhibitors of interest are specially those cited in examples 1 to 19. Published patent application WO 9925719 discloses sulphostin, a DPP-IV inhibitor prepared by culturing a Streptomyces microorganism. WO 9938501 discloses N-substituted 4- to 8-membered heterocyclic rings. DPP-IV inhibitors of interest are specially those cited in claims 15 to 20.

WO 9946272 discloses phosphoric compounds as inhibitors of DPP-IV. DPP-IV inhibitors of interest are specially those cited in claims 1 to 23.

Other preferred DPP-IV inhibitors are the compounds of formula I, II or III disclosed in the patent application WO 03/057200 on page 14 to 27. DPP-IV inhibitors are the compounds specifically described on pages 28 and 29.

Published patent applications WO 9967278 and WO 9967279 disclose DPP-IV prodrugs and inhibitors of the form A-B-C where C is either a stable or unstable inhibitor of DPP-IV.

Known DPP-IV inhibitors are compounds of formula (IA) or (IB) 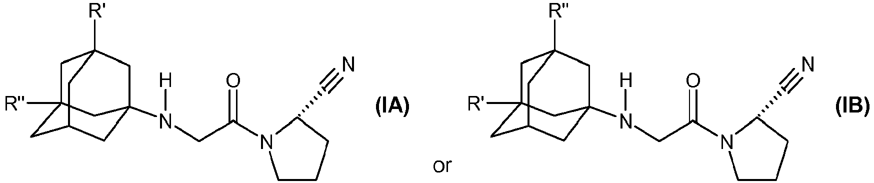 wherein
R' represents hydroxy, C₁-C₇alkoxy, C₁-C₈alkanoyloxy or R₅R₄N-CO-O-, where R₄ and R₅ independently are C₁-C₇alkyl or phenyl which is unsubstituted or substituted by a substitutent selected from C₁-C₇alkyl, C₁-C₇alkoxy, halogen and trifluoromethyl and where R₄ additionally is hydrogen; or R₄ and R₅ together represent C₃-C₆alkylene; and
R" represents hydrogen; or
R' and R" independently represent C₁-C₇alkyl;
in free form or in form of a pharmaceutically acceptable acid addition salt.

These DPP-IV inhibitor compounds of formula (I), (IA) or (IB) are known and described in U.S. Patent No. 6,166,063, issued December 26, 2000 and WO 01/52825. Specially disclosed is (S)-1-{2-[5-cyanopyridin-2yl)amino]ethyl-aminoacetyl)-2-cyano-pyrrolidine or (S)-1-[(3-hydroxy-1 adamantyl)amino]acetyl-2-cyano-pyrrolidine (LAF237). They can exist in free form or in acid addition salt form. Pharmaceutically acceptable, i.e., non-toxic and physiologically acceptable, salts are useful, although other salts are also useful, e.g., in isolating or purifying the compounds Although the preferred acid addition salts are the hydrochlorides, salts of methanesulfonic, sulfuric, phosphoric, citric, lactic and acetic acid may also be utilized.

DPP-IV inhibitors are those described by Mona Patel and col. (Expert Opinion Investig Drugs. 2003 Apr;12(4):623-33) on the paragraph 5, especially P32/98, K-364, FE-999011, BDPX, NVP-DDP-728 and others.

FE-999011 is described in the patent application WO 95/15309 page 14, as compound No. 18.

Another inhibitor is the compound BMS-477118 disclosed in U.S. Patent No. 6,395,767 (compound of example 60) also known as is (1S,3S,5S)-2-[(2S)-2-amino-2-(3-hydroxytricyclo[3.3.1.1^{3,7}]dec-1-yl)-1-oxoethyl]-2-azabicyclo[3.1.0]hexane-3-carbonitrile, benzoate (1:1) as depicted in Formula M of the patent application WO 2004/052850 on page 2, and the corresponding free base, (IS,3S,5S)-2-[(2S)-2-amino-2-(3-hydroxy-tricyclo[3.3.1.1^{3,7}]dec-1-yl)-1-oxoethyl]-2-azabicyclo-[3.1.0]hexane-3-carbonitrile (M') and its monohydrate (M") as depicted in Formula M of the patent application WO 2004/052850 on page 3.
Another inhibitor is the compound GSK23A disclosed in WO 03/002531 (example 9) also known as (2S,4S)- 1- ((2R)-2-Amino-3-[(4-methoxybenzyl)sulfonyl]-3-methylbutanoyl)-4-fluoropyrrolidine-2-carbonitrile hydrochloride.

Other DPP-IV inhibitors are described in the International patent application WO 02/076450 (especially the examples 1 to 128) and by Wallace T. Ashton (Bioorganic & Medicinal Chemistry Letters 14 (2004) 859-863) especially the compound 1 and the compounds listed in the tables 1 and 2. P32/98 or P3298 (CAS number: 251572-86-8) also known as 3-[(2S,3S)-2-amino-3-methyl-1-oxopentyl]thiazolidine can be used as 3-[(2S,3S)-2-amino-3-methyl-1-oxopentyl]thiazolidine and (2E)-2-butenedioate (2:1) mixture such as shown below 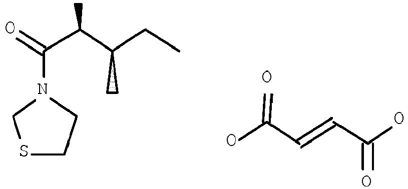 and is described in WO 99/61431 in the name of Probiodrug and also the compound P 93/01.

Other DPP-IV inhibitors are the compounds disclosed in the patent application WO 02/083128 such as in the claims 1 to 5. DPP-IV inhibitors are the compounds specifically described by the examples 1 to 13 and the claims 6 to 10.

Other DPP-IV inhibitors are described in the patent applications WO 2004/037169 especially those described in the examples 1 to 48 and WO 02/062764 especially the described examples 1 to 293, compounds 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide described on page 7 and also in the patent application WO2004/024184 especially in the reference examples 1 to 4.

Other DPP-IV inhibitors are described in the patent application WO 03/004498 especially examples 1 to 33 and the compound of the formula 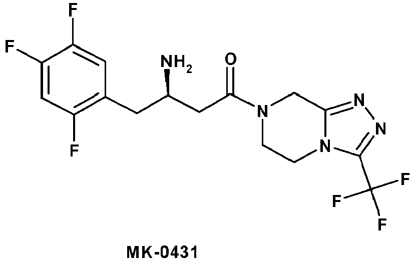 described by the example 7 and also known as MK-0431.

DPP-IV inhibitors are also described in the patent application WO 2004/037181 especially examples 1 to 33, the compounds described in the claims 3 to 5. DPP-IV inhibitors are N-substituted adamantyl-amino- acetyl-2-cyano pyrrolidines, N (substituted glycyl)-4-cyano pyrrolidines, N- (N'-substituted glycyl)-2-cyanopyrrolidines, N-aminoacyl thiazolidines, N-aminoacyl pyrrolidines, L-allo-isoleucyl thiazolidine, L-threo-isoleucyl pyrrolidine, and L-allo-isoleucyl pyrrolidine, 1-[2-[(5-cyanopyridin-2-yl) amino] ethylamino] acetyl-2-cyano- (S)-pyrrolidine and pharmaceutical salts thereof. Other known inhibitors are 1-{2-[(5-cyanopyridin-2-yl)amino]ethylamino}acetyl-2(S)-cyanopyrrolidine dihydrochloride (DPP728), of formula 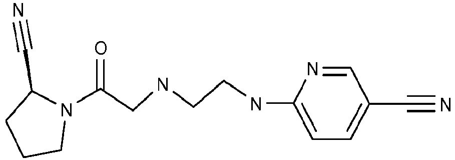 especially the dihydrochloride thereof,
and (S)-1-[(3-hydroxy-1-adamantyl)amino]acetyl-2-cyano-pyrrolidine (LAF237) of formula 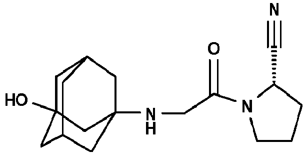 and L-threo-isoleucyl thiazolidine (compound code according to Probiodrug: P32/98 as described above), MK-0431, GSK23A, BMS-477118, 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide and optionally in any case pharmaceutical salts thereof.

DPP728 and LAF237 are specifically disclosed in Example 3 of WO 98/19998 and Example 1 of WO 00/34241, respectively. The DPP-IV inhibitor P32/98 (see above) is specifically described in Diabetes 1998, 47, 1253-1258. DPP728 and LAF237 can be formulated as described on page 20 of WO 98/19998 or in WO 00/34241. Formulations for the administration of LAF237 are described in the US provisional application No. 60/604274.

The DPP-IV inhibitor LAF237 may be administered in enantiomerically pure form, e.g., >98%, preferably >99%; or together with the R enantiomer, e.g., in racemic form. The above dosage ranges are based on the compounds of formula (I), excluding the amount of the R enantiomer.

In view of its ability to inhibit DPP-IV, the DPP-IV inhibitor LAF237 and its corresponding pharmaceutically acceptable acid addition salts, are useful in treating conditions mediated by DPP-IV inhibition. Based on the above and findings in the literature, it is expected that LAF237 is useful in the treatment of conditions, such as non-insulin-dependent diabetes mellitus, arthritis, obesity, allograft transplantation and calcitonin-osteoporosis. In addition, based on the roles of glucagon-like peptides, such as GLP-1 and GLP-2, and their association with DPP-IV inhibition, it is expected that LAF237 is useful for example, to produce a sedative or anxiolytic effect, or to attenuate post-surgical catabolic changes and hormonal responses to stress, or to reduce mortality and morbidity after myocardial infarction, or in the treatment of conditions related to the above effects which may be mediated by GLP-1 and/or GLP-2 levels.

More specifically, e.g., the DPP-IV inhibitor LAF237 and its corresponding pharmaceutically acceptable acid addition salts, improve early insulin response to an oral glucose challenge and, therefore, are useful in treating non-insulin-dependent diabetes mellitus.

The DPP-IV inhibitor compounds, especially compounds of formula IA or IB, are hygroscopic, present stability problems, and are not inherently compressible. Consequently, there is a need to provide a free-flowing and cohesive composition capable of being directly compressed into strong tablets with an acceptable *in vitro* dissolution profile. Tablets may be defined as solid dosage pharmaceutical forms containing drug substances with or without suitable fillers. They are produced by compression or compaction of a formulation containing the active ingredient and certain excipients selected to aid in the processing and to improve the properties of the product. Tablets may be coated or uncoated and are made from powdered, crystalline materials. They may include various diluents, binders, disintegrants, lubricants, glidants and in many cases, colorants. Excipients used are classified according to the function they perform. For example, a glidant may be used to improve the flow of powder blend in the hopper and into the tablet die.

There has been widespread use of tablets since the latter part of the 19^{th} century and the majority of pharmaceutical dosage forms are marketed as tablets. Major reasons of tablet popularity as a dosage form are simplicity, low cost and the speed of production. Other reasons include stability of drug product, convenience in packaging, shipping and dispensing. To the patient or consumer, tablets offer convenience of administration, ease of accurate dosage, compactness, portability, blandness of taste, ease of administration and elegant distinctive appearance.

Tablets may be plain, film or sugar coated bisected, embossed, layered or sustained-release. They can be made in a variety of sizes, shapes and colors. Tablets may be swallowed, chewed or dissolved in the buccal cavity or beneath the tongue. They may be dissolved in water for local or topical application. Sterile tablets are normally used for parenteral solutions and for implantation beneath the skin.

In addition to the active or therapeutic ingredients, tablets may contain a number of inert materials known as excipients. They may be classified according to the role they play in the final tablet. The primary composition includes a filler, binder, lubricant and glidant. Other excipients which give physical characteristics to the finished tablet are coloring agents, and flavors in the case of chewable tablets. Without excipients most drugs and pharmaceutical ingredients cannot be directly-compressed into tablets. This is primarily due to the poor flow and cohesive properties of most drugs. Typically, excipients are added to a formulation to impart good flow and compression characteristics to the material being compressed. Such properties are imparted to these excipients through pretreatment steps, such as wet granulation, slugging, spray drying spheronization or crystallization.

Lubricants are typically added to prevent the tableting materials from sticking to punches, minimize friction during tablet compression, and allow for removal of the compressed tablet from the die. Such lubricants are commonly included in the final tablet mix in amounts usually less than 1% by weight.

In addition, tablets often contain diluents which are added to increase the bulk weight of the blend resulting in a practical size for compression. This is often necessary where the dose of the drug is relatively small.

Another commonly used class of excipients in tablets is binders. Binders are agents, which impart cohesive qualities to the powdered material. Commonly used binders include starch, and sugars, such as sucrose, glucose, dextrose and lactose.

Disintegrants are often included to ensure that the tablet has an acceptable rate of disintegration. Typical disintegrants include starch derivatives and salts of carboxymethylcellulose.

Other desirable characteristics of excipients include the following:
- High-compressibility to allow strong tablets to be made at low compression forces;
- Good flow properties that can improve the flow of other excipients in the formula; and
- Cohesiveness (to prevent tablet from crumbling during processing, shipping and handling).

There are three commercially important processes for making compressed tablets: wet granulation, direct compression and dry granulation (slugging or roller compaction). The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets. Preformulation studies are done to determine the chemical and physical compatibility of the active component with proposed excipients.

The properties of the drug, its dosage forms and the economics of the operation will determine selection of the best process for tableting. Generally, both wet granulation and direct compression are used in developing a tablet.

The dry granulation method may be used where one of the constituents, either the drug or the diluent, has sufficient cohesive properties to be tabletted. The method consists of blending, slugging the ingredients, dry screening, lubrication and compression.

The wet granulation method is used to convert a powder mixture into granules having suitable flow and cohesive properties for tableting. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes weighing, dry powder blending, wet granulating, drying, milling, blending lubrication and compression.

In general, powders do not have sufficient adhesive or cohesive properties to form hard, strong granules. A binder is usually required to bond the powder particles together due to the poor cohesive properties of most powders. Heat and moisture sensitive drugs cannot usually be manufactured using wet granulation. The large number of processing steps and processing time are problems due to high level manufacturing costs. Wet granulation has also been known to reduce the compressibility of some pharmaceutical excipients, such as microcrystalline cellulose.

Direct compression is regarded as a relatively quick process where the powdered materials are compressed directly without changing the physical and chemical properties of the drug. The active ingredient(s), direct compression excipients and other auxiliary substances, such as a glidant and lubricant are blended in a twin shell blender or similar low shear apparatus before being compressed into tablets. This type of mixing was believed to be essential in order to prepare "pharmaceutically acceptable" dosage forms. Some pharmaceutical scientists believe that the manner in which a lubricant is added to a formulation must be carefully controlled. Accordingly, lubricants are usually added to a granulation by gentle mixing. It is also believed that prolonged blending of a lubricant with a granulation can materially affect hardness and disintegration time for the resulting tablets. Excessive blending of lubricants with the granulate ingredients can cause water proofing of the granule and reduces tablet hardness or strength of the compressed tablet. For these reasons, high-shear mixing conditions have not been used to prepare direct compression dosage forms.

The advantages of direct compression include uniformity of blend, few manufacturing steps involved, i.e., the overall process involves weighing of powders, blending and compression, hence less cost; elimination of heat and moisture, prime particle dissociation and physical stability.

Pharmaceutical manufacturers would prefer to use direct compression techniques over wet or dry granulation methods because of quick processing time and cost advantages. However, direct compression is usually limited to those situations where the drug or active ingredient has physical characteristics required to form pharmaceutically acceptable tablets. However, one or more excipients must often be combined with the active ingredient before the direct-compression method can be used since many ingredients do not have the necessary properties. Since each excipient added to the formulation increases the tablet size of the final product, manufacturers are often limited to using the direct-compression method in formulations containing a low dose of the active ingredient per compressed tablet.

A solid dosage form containing a high-dose drug, i.e., the drug itself comprises a substantial portion of the total compressed tablet weight, could only be directly compressed if the drug itself has sufficient physical characteristics, e.g., cohesiveness, for the ingredients to be directly compressed.

For an example, the DPP-IV inhibitor e.g. those of formula (I) is considered a high-dose drug. Most tablet formulations include a range of 70-85% by weight of DPP-IV inhibitor per tablet. This high-dose drug, combined with its rather poor physical characteristics for direct compression, has not permitted direct compression as a method to prepare the final tablet. In addition, the active ingredients have poor stability in presence of water, another factor militating against the use of the wet granulation method.

Another limitation of direct compression as a method of tablet manufacturing is the potential size of the compressed tablets. If the amount of active ingredient is high, a pharmaceutical formulator may choose to wet granulate the active ingredient with other excipients to attain an acceptable sized tablet with the desired amount of active ingredient. The amount of filler, binder or other excipients needed in wet granulation is less than that required for direct compression since the process of wet granulation contributes toward the desired physical properties of the tablet.

Hydroxypropyl methylcellulose has been utilized in the pharmaceutical industry as a direct compression excipient for solid dose forms. Hydroxypropyl methylcellulose is a processed cellulose and controls drug release from solid dosage forms.

Despite the advantages of the direct compression, such as reduced processing time and cost, wet granulation is widely-used in the industry to prepare solid dosage forms. Wet granulation is often preferred over direct compression because wet granulation has a greater chance of overcoming any problems associated with the physical characteristics of various ingredients in the formulation. This provides material which has the required flow and cohesive properties necessary to obtain an acceptable solid dosage form.

The popularity of wet granulation compared to direct compression is based on at least three advantages. First, wet granulation provides the material to be compressed with better wetting properties, particularly in the case of hydrophobic drug substances. The addition of hydrophilic excipients makes the surface of the hydrophobic drug more hydrophilic, reducing disintegration and dissolution problems. Second, the content uniformity of the solid dosage form is generally improved with wet granulation because all of the granules usually contain the same amount of drug. Lastly, the segregation of drug(s) from excipients is avoided.

Segregation could be a potential problem with direct compression. The size and shape of particles comprising the granulate to be compressed are optimized through the wet granulation process. This is because when a dry solid is wet granulated the binder "glues" particles together, so that they agglomerate into spherical granules.

In spite of the advantages afforded by wet granulation in general, due to the instability of the compounds in the presence of water, it is desirable to directly compress tablets containing high-dose DPP-IV inhibitor, e.g. as that defined in formula (I). There is a need in the industry for techniques and pharmaceutical excipients which will allow manufacturers to prepare high-dose DPP-IV inhibitor tablets by direct compression.

It is an object of the invention to provide a direct compressed DPP-IV inhibitor LAF237 tablet in unit dosage form having an acceptable dissolution profile, as well as acceptable degrees of hardness and resistance to chipping, as well as a short disintegration time.

It is a further object of the invention to provide a process for preparing a compressed DPP-IV inhibitor LAF237 tablet by direct compression in unit dosage form.

In addition to the active ingredient, the tableting powder contains a number of inert materials known as excipients. They may be classified according to the role they play in the final tablet. The primary composition includes fillers, binders or diluents, lubricants, disintegrants and glidants. Other excipients which give physical characteristics to the finished tablet are coloring agents, and flavors in the case of chewable tablets. Typically, excipients are added to a formulation to impart good flow and compression characteristics to the material being compressed.

The formulation of this invention is defined in the independent claim 1 and comprises the following: the active ingredient which is the DPP-IV inhibitor LAF237, the binders or diluents which are microcrystalline cellulose and lactose, the disintegrant which is sodium starch glycolate and the lubricant which is magnesium stearate. The diluent microcrystalline cellulose is manufactured by the controlled hydrolysis of alpha-cellulose, obtained as a pulp from fibrous plant materials, with dilute mineral acid solutions. Following hydrolysis, the hydrocellulose is purified by filtration and the aqueous slurry is spray dried to form dry, porous particles of a broad size distribution. Suitable microcrystalline cellulose will have an average particle size of from about 20 nm to about 200 nm. Microcrystalline cellulose is available from several suppliers. Suitable microcrystalline cellulose includes Avicel PH 101, Avicel PH 102, Avicel PH 103, Avicel PH 105 and Avicel PH 200, manufactured by FMC Corporation. Particularly preferred in the practice of this invention is Avicel PH 102, which has the smallest surface area and pore structure. Preferably the microcrystalline cellulose is present in a tablet formulation in an amount of from about 30% to about 35% by weight; yet another preferred range of from about 30% to about 32% by weight.

Another diluent is lactose. Preferably, the lactose is ground to have an average particle size of between about 50 µm and about 500 µm prior to formulating. The lactose is present in the tablet formulation in an amount of from 20% to 25% by weight.

Magnesium stearate reduces the friction between the die wall and
tablet mix during the compression and ejection of the tablets. It helps prevent adhesion of tablets to the punches and dies. Magnesium stearate also aids in the flow of the powder in the hopper and into the die. It has a particle size range of 450-550 microns and a density range of 1.00-1.80 g/mL. It is stable and does not polymerize within the tableting mix. Magnesium stearate is also employed in the formulation, in an amount from 0.1% to 2% by weight.

Composition as described above comprising between 20 and 120 mg of LAF237 preferably between 25 and 100m of LAF237 or a pharmaceutically acceptable acid addition salt thereof.

Most preferably the above described compositions comprise two diluents selected from microcrystalline cellulose such as Avicel PH 102 and lactose.

Still another preferred composition is the following:
(a) from 22% to 28% preferably 24-26% by weight on a dry weight basis of the DPP-IV inhibitor LAF237;
(b) from 45% to 50% by weight on a dry weight basis of a pharmaceutically acceptable microcrystalline cellulose;
(c) from 20% to 25% by weight on a dry weight basis of a pharmaceutically acceptable lactose;
(d) from 1.5% to 2.5% by weight on a dry weight basis of a pharmaceutically acceptable sodium starch glycolate; and
(e) from 0.1% to 2% by weight on a dry weight basis of magnesium stearate.

Still another preferred composition is the following:
(a) from 24-26% by weight on a dry weight basis of the DPP-IV inhibitor LAF237;
(b) from about 46% to about 48% by weight on a dry weight basis of a pharmaceutically acceptable microcrystalline cellulose;
(c) from about 23% to about 24.5% by weight on a dry weight basis of a pharmaceutically acceptable lactose;
(d) from 1.5% to 2.5% by weight on a dry weight basis of a pharmaceutically acceptable sodium starch glycolate; and
(e) from 0.1% to 2% by weight on a dry weight basis of magnesium stearate.

Additional conventional excipients can optionally be added to the herein described formulations such as the conventional solid fillers or carriers described hereinabove. The above described formulations are particularly adapted for the production of pharmaceutical tablets which are direct compressed tablets, and provides the necessary physical characteristics, dissolution and drug release profiles as required by one of ordinary necessary physical skill in the art. Therefore in an additional embodiment, the present invention concerns the use of any of the above described formulations, for the manufacture of pharmaceutical tablets, by direct compression. In particular the tablets obtained with the above described formulations especially when processed in the form of direct compressed tablets or the below described direct compressed tablets, have very low friability problems, very good breaking strength, improved manufacturing robustness, optimal tablet thickness to tablet weight ratios (direct compressed tablets), less water in the formulation especially directed compressed tablet, good Dispersion Disintegration time DT according to the British Pharmacopoeia 1988, good Dispersion Quality.

This present invention of direct compression of DPP-IV inhibitor involves blending and compression. The choice of grades of excipients took into consideration particle size maintained within a range that allows homogeneity of the powder mix and content uniformity of DPP-IV inhibitor. It prevents segregation of powders in the hopper during direct compression. The advantages of using these excipients are that they impart compressibility, cohesiveness and flowability of the powder blend. In addition, the use of direct compression provides competitive unit production cost, shelf life, eliminates heat and moisture, allows for prime particle dissociation, physical stability and ensures particle size uniformity.

The described advantages of the claimed compositions are also very useful for e.g. roller compaction or wet granulation or to fill capsules.

In the development of the herein described pharmaceutical compositions, the applicant has discovered that the direct compressed tablet is particularly advantageous if;
i) the particles comprising the DPP-IV inhibitor have a particle size distribution of between 10 to 250 µm, and/or
ii) the water content of the tablet at less than 10% after 1 week at 25°C and 60% room humidity (RH), and/or
iii) tablet thickness to tablet weight ratios is of 0.002 to 0.06 mm/mg.

Thus, the present invention concerns a direct compressed pharmaceutical tablet, comprising a DPP-IV inhibitor, in free form or in acid addition salt form, having physical properties that render the tableting into direct compressed pharmaceutical tablet unlikely or very difficult. The DPP-IV inhibitor is LAF237.

Thus in a first embodiment (a), the present invention concerns direct compressed pharmaceutical tablets, wherein the dispersion contains particles comprising DPP-IV inhibitor LAF237, in free form or in acid addition salt form, and wherein at least 80% and most preferably 90% of the particle size distribution of the particles comprising (S)-1-[(3-hydroxy-1-adamantyl)amino]acetyl-2-cyanopyrrolidine in free form or in acid addition salt form is between 10 to 250 µm and wherein at least 35% and most preferably 45% of the particle size distribution of the particles comprising (S)-1-[(3-hydroxy-1-adamantyl)amino]acetyl-2-cyanopyrrolidine in free form or in acid addition salt form is between 50 to 150 µm.

In a second embodiment (b), this invention concerns a direct compressed pharmaceutical tablet wherein the dispersion contains particles comprising DPP-IV inhibitor LAF237, in free form or in acid addition salt form, and wherein tablet thickness to tablet weight ratios is of 0.002 to 0.06 mm/mg preferably of 0.01 to 0.03 mm/mg.

The combination of the above first and second embodiments (a) and (b), provide direct compressed tablets with good compaction characteristics.

Thus this invention concerns also a direct compressed tablet wherein the dispersion contains particles comprising DPP-IV inhibitor LAF237, in free form or in acid addition salt form, and wherein;
i) at least 80% and most preferably 90% of the particle size distribution of the particles comprising (S)-1-[(3-hydroxy-1-adamantyl)amino]acetyl-2-cyanopyrrolidine in free form or in acid addition salt form is between 10 to 250 µm and wherein at least 35% of the particle size distribution of the particles comprising (S)-1-[(3-hydroxy-1-adamantyl)amino]acetyl-2-cyanopyrrolidine in free form or in acid addition salt form is between 50 to 150 µm, and
ii) tablet thickness to tablet weight ratios is of 0.002 to 0.06 mm/mg or of 0.01 to 0.03 mm/mg
preferably wherein;
i) at least 45% of the particle size distribution of the particles comprising (S)-1-[(3-hydroxy-1-adamantyl)amino]acetyl-2-cyanopyrrolidine in free form or in acid addition salt form is between 50 to 150 µm, and
ii) tablet thickness to tablet weight ratios is of 0.002 to 0.06 mm/mg or of 0.01 to 0.03 mm/mg.

In a third embodiment, this invention concerns a direct compressed pharmaceutical tablet wherein the dispersion contains particles comprising DPP-IV inhibitor LAF237, in free form or in acid addition salt form, and wherein;
i) at least 80% and most preferably 90% of the particle size distribution of the particles comprising (S)-1-[(3-hydroxy-1-adamantyl)amino]acetyl-2-cyanopyrrolidine in free form or in acid addition salt form is between 10 to 250 µm and wherein at least 35% of the particle size distribution of the particles comprising (S)-1-[(3-hydroxy-1-adamantyl)amino]acetyl-2-cyanopyrrolidine in free form or in acid addition salt form is between 50 to 150 µm,
ii) the water content of the tablet is less than 10% after 1 week at 25°C and 60% RH, and
iii) tablet thickness to tablet weight ratios is of 0.002 to 0.06 mm/mg.

Preferably this invention concerns a direct compressed tablet wherein the dispersion contains particles comprising DPP-IV inhibitor LAF237, in free form or in acid addition salt form, and wherein;
i) at least 35% and most preferably 45% of the particle size distribution of the particles comprising (S)-1-[(3-hydroxy-1-adamantyl)amino]acetyl-2-cyanopyrrolidine in free form or in acid addition salt form is between 50 to 150 µm,
ii) the water content of the tablet is less than 10% after 1 week at 25°C and 60% RH, and
iii) tablet thickness to tablet weight ratios is of 0.002 to 0.06 mm/mg.

Preferably this invention concerns a direct compressed tablet wherein the dispersion contains particles comprising DPP-IV inhibitor LAF237, in free form or in acid addition salt form, and wherein;
i) at least 35% and most preferably 45% of the particle size distribution of the particles comprising (S)-1-[(3-hydroxy-1-adamantyl)amino]acetyl-2-cyanopyrrolidine in free form or in acid addition salt form is between 50 to 150 µm,
ii) the water content of the tablet is less than 5% after 1 week at 25°C and 60% RH, and
iii) tablet thickness to tablet weight ratios is of 0.002 to 0.06 mm/mg.

Preferably this invention concerns a direct compressed tablet wherein the dispersion contains particles comprising DPP-IV inhibitor LAF237, in free form or in acid addition salt form, and wherein;
i) at least 35% and most preferably 45% of the particle size distribution of the particles comprising (S)-1-[(3-hydroxy-1-adamantyl)amino]acetyl-2-cyanopyrrolidine in free form or in acid addition salt form is between 50 to 150 µm,
ii) the water content of the tablet is less than 5% after 1 week at 25°C and 60% RH, and
iii) tablet thickness to tablet weight ratios is of 0.01 to 0.03 mm/mg

Preferably the LAF237 particles comprise more than 70% of DPPIV inhibitor, most preferably more than 90% or 95% and even more preferably more than 98% of DPPIV inhibitor.

Preferably the LAF237 particles comprise more than 70% of LAF237, most preferably more than 90% or 95% and even more preferably more than 98% of LAF237.

It has been discovered that the selected particle size distribution of DPPIV inhibitor

LAF237 were particularly important to provide the best compaction of the tablets.

In an additional preferred embodiment, the particle size distribution of the selected excipients (b), (c) and/or (d) is similar to the particle size distribution of the DPP-IV inhibitor LAF237 particles.

The term "similar", means that the particle size distribution of the excipient in the tablet is between 5 and 400µm, or between 10 and 300 µm, preferably between 10 to 250 µm.

The preferred excipients with an adapted particle size distribution can be picked from e.g. Handbook of Pharmaceutical Excipients (4th edition), Edited by Raymond C Rowe - Publisher: Science and Practice.

Particle size of drug LAF237 particles size, is controlled by crystallisazion, drying and/or milling/sieving (non limiting examples are described below). Particle size can also be comminuted using roller compaction and milling/sieving. Producing the right particle size is well known and described in the art such as in "Pharmaceutical dosage forms: volume 2, 2nd edition, Ed.: H.A. Lieberman, L. Lachman, J.B. Schwarz (Chapter 3: SIZE REDUCTION)". Multiple particle sizes have been studied and it has been discovered that the herein described specific size range provides unexpected good results for direct compaction.

PARTICLE SIZE DISTRIBUTION is measured by laser diffraction (international standard ISO 13320-1)

Water content of the tablet can be measured using Loss on drying method or Karl-Fischer method which are well known methods to the person skilled in the art (e.g. water content can be measured by loss on drying by thermo-grametry). Such methods are well known and described in the art such as in any analytical chemistry text book (J.A. Dean, Analytical Chemistry Handbook, Section 19, McGraw-Hill, New York, 1995) or by the United State Pharmacopeia's (USP) publication USP-NF (2004) which describes the US Food and Drug Administration (FDA) enforceable standards ((2004 - USP - Chapter 921).

Tablet thickness is measurable using a ruler, vernier caliper, a screw gauge or any electronic method to measure dimensions. We take the tablet thickness in mm and divide by tablet weight in mg to get the ratio. Such methods are well known and described in the art such as in any analytical chemistry text book or by the United State Pharmacopeia's (USP) publication USP-NF (2004) which describes the US Food and Drug Administration (FDA) enforceable standards.

This invention provides in particular a direct compressed tablet which is capable of dispersing in water within a period of 5 to 15 minutes to provide a dispersion which is capable of passing through a sieve screen with a mesh aperture of 710 µm in accordance with the herein defined British Pharmacopoeia test for dispersible tablets.

A tablet according to the invention, as well as being quickly dispersible in water, has the added advantage that it meets the British Pharmacopoeia (B.P.) test for dispersible tablets in respect of dispersion times and dispersion quality (i.e. passage through a 710 µm sieve). Preferably the dispersion time of a tablet according to the invention is less than 15 minutes, more preferably less than 12 minutes and most preferably less than 10 minute.

A further advantage of the tablets according to invention is that because a relatively fine dispersion is formed the tablet will have a lower dissolution time and thus the drug may be absorbed into the blood stream much faster. Furthermore the fast dispersion times and relatively fine dispersions obtained with tablets according to the invention are also advantageous for swallowable tablets. Thus tablets according to the invention can be presented both for dispersion in water and also for directly swallowing. Those tablets according to the invention that are intended for swelling are preferably film-coated to aid swallowing.

In a further embodiment the present invention concerns a direct compressed tablet with improved dissolution rates (dissolution of the drug), wherein the dispersion contains particles i.e. LAF237 particles comprising LAF237, in free form or in acid addition salt form, wherein at least 80% and most preferably 90% of the particle size distribution in the tablet is between 10 to 250 mm,
and wherein
i) between 0 and 10 minutes 85 to 99.5 % of the active ingredient is released, and
ii) between 10 and 15 minutes 90 to 99.5 % of the active ingredient is released,
preferably wherein,
i) between 0 and 10 minutes 88 to 99.5 % of the active ingredient is released, and
ii) between 10 and 15 minutes 95 to 99.5 % of the active ingredient is released,
or preferably
i) between 0 and 10 minutes 89 to 94 % of the active ingredient is released, and
ii) between 10 and 15 minutes 96 to 99 % of the active ingredient is released

The Paddle method to measure the drug dissolution rate (% of release) is used with 1000ml of 0.01N HCl. Such methods are well known and described in the art such as in any analytical chemistry text book or by the United State Pharmacopeia's (USP) publication USP-NF (2004 - Chapter 711) which describes the US Food and Drug Administration (FDA) enforceable standards.

The invention also provides a process for preparing a compressed DPP-IV inhibitor tablet in unit dosage form, wherein;
i) at least 80% and most preferably 90% of the particles comprising DPP-IV inhibitor LAF237, in free form or in acid addition salt form, in the tablet have a particle size distribution of between 10 to 250 µm and wherein at least 35% of the particle size distribution of the particles comprising (S)-1-[(3-hydroxy-1-adamantyl)amino]acetyl-2-cyanopyrrolidine in free form or in acid addition salt form is between 50 to 150 µm,
ii) the water content of the tablet is less than 10% after 1 week at 25°C and 60% RH, and
iii) tablet thickness to tablet weight ratios is of 0.002 to 0.06 mm
which comprises:
(a) blending as a % by weight on a dry weight basis the components as defined in the claims to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

The invention also provides a process for preparing a compressed DPP-IV inhibitor tablet in unit dosage form which comprises:
(a) blending as a % by weight on a dry weight basis the components as defined in the claims to form a DPP-IV inhibitor formulation in the form of a tableting powder, capable of being directly compressed into a tablet; and
(b) compressing the formulation prepared during step (a) to form the compressed DPP-IV inhibitor tablet in unit dosage form.

Before the compression step (b) a sieving step is preferably applied to the formulation for basic delumping i.e. to get rid of any agglomerates/cakes.

In a further aspect, the present invention concerns the use of the herein described direct compressed tablets for the treatment of conditions, such as non-insulin-dependent diabetes mellitus, arthritis, obesity, allograft transplantation, calcitonin-osteoporosis, Heart Failure, Impaired Glucose Metabolism), IGT (Impaired Glucose Tolerance), neurodegenerative diseases such as Alzheimer's and Parkinson disease, modulating hyperlipidemia, modulating conditions associated with hyperlipidemia or for lowering VLDL, LDL and Lp(a) levels, cardiovascular or renal diseases e.g. diabetic cardiomyopathy, left or right ventricular hypertrophy, hypertrophic medial thickening in arteries and/or in large vessels, mesenteric vasculature hypertrophy, mesanglial hypertrophy, neurodegenerative disorders and cognitive disorders, to produce a sedative or anxiolytic effect, to attenuate post-surgical catabolic changes and hormonal responses to stress, to reduce mortality and morbidity after myocardial infarction, the treatment of conditions related to the above effects which may be mediated by GLP-1 and/or GLP-2 levels.

This invention is further illustrated by the following example:

### Example 1 (not according to the invention)

To prepare the 25 mg tablet size (directly compressed tablet), a batch size of 7 kg is prepared using amounts corresponding to the following per unit: 25 mg per unit of the compound 1-[3-hydroxy-adamant-1-ylamino)-acetyl]-pyrrolidine-2(*S*)-carbonitrile is mixed with 35.1 mg of microcrystalline cellulose, 17.5 mg anhydrous lactose and 1.6 mg sodium starch glycolate. The ingredients are pre-blended together in a commercial bin blender, then sieved through a 500 µm or 850 µm screen. The mix is blended again in the bin blender, then the necessary amount of the magnesium stearate to yield the 0.8 mg magnesium stearate per 25 mg tablet size, is added. Blending in each step is conducted at about 150-450 rotations, to ensure homogeneity of the mixture. Following blending again in the bin blender, the mix can be tabletted in a conventional tableting machine. The individual tablet weight for the 25 mg tablet is 80 mg. Tablets having 50 mg active ingredient weigh 160 mg, and 100 mg active ingredient tablets weigh 320 mg, respectively. The blend is a powder which has excellent compressibility into the desired tablet size.

### Example 2

The same process as described above in example 1, can be applied to produce the below described preferred 50 mg tablet (directly compressed).

| **Components** | **Composition per unit (mg)** | **Quantity per batch (kg)** |
|---|---|---|
| LAF 237 drug substance | 50.00 | 65.0 |
| Microcrystalline cellulose, PH102 (Ph.Eur., NF) | 95.68 | 124.38 |
| Lactose anhydrous DT (USP, Ph.Eur.) | 47.82 | 62.17 |
| Sodium starch glycolate (USP, Ph.Eur.) | 4.00 | 5.2 |
| Magnesium stearate (Ph.Eur, NF) | 2.50 | 3.25 |
| Total weight, per tablet or per batch | 200.0 | 260.0 |

### Example 3:

The tablets prepared in accordance with the above Description and examples can be tested as follows.

### Tablet Evaluation Methods

1. Average tablet weight. Twenty tablets are weighed on an analytical balance and the average tablet weight calculated.
2. Tablet breaking strength (kilo bond-kp). 5 tablets are individually tested using a Schleuniger crushing strength tester, and the average breaking strength calculated.
3. Friability (% loss). 10 tablets, accurately weighed, are subjected to 10 minutes friability testing using a Roche Friabilator. The tablets are dedusted, reweighed, and the weight loss due to the friability is calculated as a percentage of the initial weight.
4. Dispersion Disintegration time DT (The test for dispersible tablets defined in the British Pharmacopoeia, 1988, Volume II, page 895 - BP 1988). 6 tablets are tested in accordance to the above-defined BP test (without discs) for dispersible tablets. This utilizes water at a temperature of 19°- 21° C.
5. Dispersion Quality. In accordance with the BP uniformity of dispersion test for dispersible tablets (BP 1988 Volume II page 895), two tablets are placed in 100 ml of water at 19°-21° C. and allowed to disperse.

### Granule Evaluation Methods

1. Loss on Drying (LOD). The residual moisture content of the granule (LOD) can be determined on a 3-4 g sample using a Computrac moisture analyser set at 90° C. operated in accordance with the manufacturer's procedure.
2. Weight Median Diameter (WMD). A 10 g sample of granule is sifted for 2 minutes at suitable pulse and sift amplitudes in an Allen Bradley sonic sifter in accordance with manufacturer's instructions. Sieves of 300 µm, 250 µm, 200 µm, 150 µm, 100 µm, 53 µm and 40 µm are used. The WMD is calculated from the cumulative percentage undersize size distribution using a computer program.

### Example 4:

### Improved manufacturing robustness

A preliminary compactibility assessment is carried out on a Carver press using different formulations as well as binary mixtures of LAF 237 with different excipients e.g. microcrystalline cellulose (Avicel PH102).

Data demonstrate that our claimed compositions on being compressed with increasing levels of pressure (compression force) show a substantially useful increase in tablet strength. In particular e.g. mixture of LAF237 and Avicel show a substantially useful increase in tablet strength. These results indicated that from compactibility point of view microcrystalline cellulose e.g. Avicel would a preferred excipient to be combined with LAF237. With increasing pressure (compression force) our claimed formulations and selected ranges show a substantially useful increase in tablet strength.

A compactibility study (D. Becker, personal communication) is carried out on an instrumented Korsch single station press with force and displacement sensors on both upper and lower punches.

A clear indication is afforded from these data that LAF237 tablets are very likely to have poor tablet hardness/crushing strength unless diluted out using sufficient filler with excellent compactibility. Our claimed formulations and selected ranges are particularly adapted to provide the required compactibility. Microcrystalline cellulose e.g. Avicel is a good choice for a filler in this respect.

### Example 5: Friability

Evaluation is carried out using a Manesty Betapress at 6 different settings: strain rate settings of 66-90 rpm (63,000-86,000 TPH) and force of 7.5-15 kN. The trials uses Flat-faced

Beveled-edge (FFBE) tooling of 9 mm diameter for 250 mg tablets and 10 mm diameter for 310 mg tablets (other diameters are used depending on the weight of the tested tablet). Total tablet weights were selected so that both the 9 and 10 mm FFBE tablets would have 100 mg of LAF237 and identical tablet thickness. Friability, Compression profile, Strain rate profile and Weight variation are the measured outcomes. Study design and the friability results obtained from the study are used to determine the variables (particle size distribution in the formulation, tablet weight, tablet thickness and weight, water content in the tablet etc) impacting the outcome of hardness.

### Example 6: Mechanical stress (particle size distribution)

The material in the desired particle size range can be produced from any form of vildagliptin e.g. amorphous vildagliptin, by mechanical stress. This stress can be mediated by impact, shear or compression. In most commercially available grinding equipment a combination of these principles occurs. For vildagliptin preferably a mechanical impact or jet mill is used. The most preferable mechanical impact mill can be equipped with different kind of beaters, screens, liners or with pin plates. For our process preferably an impact mill with plate beater and a slit screen 5 * 2.5 cm is used. The impact speed should be variable between 20 and 100 m/s (as peripheral speed) to adapt to any batch to batch variation. In our case a peripheral speed of the beater of about 40 - 50 m/s is used.

## Claims

1. A direct compressed pharmaceutical tablet, wherein the tablet comprises:
a) from 22% to 28% by weight on a dry weight basis of a DPP-IV inhibitor which is (S)-1-[(3-hydroxy-1-adamantyl)amino]acetyl-2-cyanopyrrolidine in free form or in acid addition salt form;
b) from 45% to 50% by weight on a dry weight basis of a pharmaceutically acceptable microcrystalline cellulose;
c) from 20% to 25% by weight on a dry weight basis of a pharmaceutically acceptable lactose;
d) from 1.5% to 2.5% by weight on a dry weight basis of a pharmaceutically acceptable sodium starch glycolate; and
e) from 0.1% to 2% by weight on a dry weight basis of magnesium stearate;
wherein the dispersion contains particles comprising said DPP-IV inhibitor which is (S)-1-[(3-hydroxy-1-adamantyl)amino]acetyl-2-cyanopyrrolidine in free form or in acid addition salt form, and wherein at least 80% of the particle size distribution of the particles comprising (S)-1-[(3-hydroxy-1-adamantyl)amino]acetyl-2-cyanopyrrolidine in free form or in acid addition salt form in the tablet is between 10 µm and 250 µm and at least 35% of the particle size distribution of the particles comprising (S)-1-[(3-hydroxy-1-adamantyl)amino]acetyl-2-cyanopyrrolidine in free form or in acid addition salt form in the tablet is between 50 µm and 150 µm, as determined by laser diffraction.

2. A direct compressed pharmaceutical tablet according to claim 1, wherein the tablet thickness to tablet weight ratio is of 0.002 to 0.06 mm/mg.

3. A direct compressed pharmaceutical tablet according any one of claims 1 to 2, wherein the tablet thickness to tablet weight ratio is of 0.01 to 0.03 mm/mg.

4. A direct compressed pharmaceutical tablet according to any one of claims 1 to 3, wherein at least 45% of the particle size distribution of the particles comprising (S)-1-[(3-hydroxy-1-adamantyl)amino]acetyl-2-cyanopyrrolidine in free form or in acid addition salt form in the tablet is between 50 µm and 150 µm, as determined by laser diffraction.

5. A direct compressed pharmaceutical tablet according to any one of claims 1 or 4, wherein the water content of the tablet is less than 5% after 1 week at 25°C and 60% RH.

6. A direct compressed pharmaceutical tablet according to claim 1, wherein
i) between 0 and 10 minutes 85% to 99.5% of the active ingredient is released, and
ii) between 10 and 15 minutes 90% to 99.5% of the active ingredient is released.

7. A direct compressed pharmaceutical tablet according to any of claims 1 to 6, wherein the particle size distribution of the pharmaceutical excipients in the tablet is between 5 and 400 µm, preferably between 10 to 250 µm, as determined by laser diffraction.

8. Process for preparing a compressed tablet according to any of claims 1 to 7, in unit dosage form, which comprises:
a) blending as a % by weight on a dry weight basis:
i) 22% to 28% by weight on a dry weight basis of a DPP-IV inhibitor which is (S)-1-[(3-hydroxy-1-adamantyl)amino]acetyl-2-cyanopyrrolidine in free form or in acid addition salt form;
ii) 45% to 50% by weight on a dry weight basis of a pharmaceutically acceptable microcrystalline cellulose;
iii) 20% to 25% by weight on a dry weight basis of a pharmaceutically acceptable lactose;
iv) 1.5% to 2.5% by weight on a dry weight basis of a pharmaceutically acceptable sodium starch glycolate; and
v) from 0.1% to 2% by weight on a dry weight basis of magnesium stearate;
to form a DPP-IV formulation in the form of a tableting powder, capable of being compressed into a tablet; and
b) compressing the formulation prepared during step a) to form the compressed DPP-IV inhibitor tablet in unit dosage form.

## Patentansprüche

1. Direktkomprimierte pharmazeutische Tablette, wobei die Tablette Folgendes umfasst:
a) 22% Gew.-% bis 28 Gew.-% auf Trockengewichtsbasis eines DPP-IV-Inhibitors, bei dem es sich um (S)-1-[(3-Hydroxy-1-adamantyl)amino]acetyl]-2-cyanopyrrolidin in freier Form oder in Säureadditionssalzform handelt,
b) 45 Gew.-% bis 50 Gew.-% auf Trockengewichtsbasis einer pharmazeutisch unbedenklichen mikrokristallinen Cellulose,
c) 20 Gew.-% bis 25 Gew.-% auf Trockengewichtsbasis einer pharmazeutisch unbedenklichen Lactose,
d) 1,5 Gew.-% bis 2,5 Gew.-% auf Trockengewichtsbasis eines pharmazeutisch unbedenklichen Natriumstärkeglykolats und
e) 0,1 Gew.-% bis 2 Gew.-% auf Trockengewichtsbasis an Magnesiumstearat,
wobei die Dispersion Partikel enthält, die den DPP-IV-Inhibitor umfassen, bei dem es sich um (S)-1-[(3-Hydroxy-1-adamantyl)amino]acetyl-2-cyanopyrrolidin in freier Form oder in Säureadditionssalzform handelt, und wobei mindestens 80% der Teilchengrößenverteilung der (S)-1-[(3-Hydroxy-1-adamantyl)amino]acetyl-2-cyanopyrrolidin in freier Form oder in Säureadditionssalzform umfassenden Partikel in der Tablette zwischen 10 µm und 250 µm liegt und mindestens 35% der Teilchengrößenverteilung der (S)-1-[(3-Hydroxy-1-adamantyl)amino]acetyl-2-cyanopyrrolidin in freier Form oder in Säureadditionssalzform umfassenden Partikel in der Tablette zwischen 50 µm und 150 µm, bestimmt durch Laserbeugung, liegt.

2. Direktkomprimierte pharmazeutische Tablette nach Anspruch 1, wobei das Verhältnis von Tablettendicke zu Tablettengewicht 0,002 bis 0,06 mm/mg beträgt.

3. Direktkomprimierte pharmazeutische Tablette nach einem der Ansprüche 1 bis 2, wobei das Verhältnis von Tablettendicke zu Tablettengewicht 0,01 bis 0,03 mm/mg beträgt.

4. Direktkomprimierte pharmazeutische Tablette nach einem der Ansprüche 1 bis 3, wobei mindestens 45% der Teilchengrößenverteilung der (S)-1-[(3-Hydroxy-1-adamantyl)amino]acetyl-2-cyanopyrrolidin in freier Form oder in Säureadditionssalzform umfassenden Partikel in der Tablette zwischen 50 µm und 150 µm, bestimmt durch Laserbeugung, liegt.

5. Direktkomprimierte pharmazeutische Tablette nach einem der Ansprüche 1 oder 4, wobei der Wassergehalt der Tablette nach 1 Woche bei 25°C und 60% RF weniger als 5% beträgt.

6. Direktkomprimierte pharmazeutische Tablette nach Anspruch 1, wobei
i) zwischen 0 und 10 Minuten 85% bis 99,5% des Wirkstoffs freigesetzt sind und
ii) zwischen 10 Minuten und 15 Minuten 90% bis 99,5% des Wirkstoffs freigesetzt sind.

7. Direktkomprimierte pharmazeutische Tablette nach einem der Ansprüche 1 bis 6, wobei die Teilchengrößenverteilung der pharmazeutischen Exzipienten in der Tablette zwischen 5 und 400 µm, vorzugsweise zwischen 10 und 250 µm, bestimmt durch Laserbeugung, liegt.

8. Verfahren zur Herstellung einer komprimierten Tablette nach einem der Ansprüche 1 bis 7 in Einheitsdosisform, bei dem man:
a) in Gew.-% auf Trockengewichtsbasis:
i) 22% Gew.-% bis 28 Gew.-% auf Trockengewichtsbasis eines DPP-IV-Inhibitors, bei dem es sich um (S)-1-[(3-Hydroxy-1-adamantyl)amino]acetyl]-2-cyanopyrrolidin in freier Form oder in Säureadditionssalzform handelt,
ii) 45 Gew.-% bis 50 Gew.-% auf Trockengewichtsbasis einer pharmazeutisch unbedenklichen mikrokristallinen Cellulose,
iii) 20 Gew.-% bis 25 Gew.-% auf Trockengewichtsbasis einer pharmazeutisch unbedenklichen Lactose,
iv) 1,5 Gew.-% bis 2,5 Gew.-% auf Trockengewichtsbasis eines pharmazeutisch unbedenklichen Natriumstärkeglykolats und
v) 0,1 Gew.-% bis 2 Gew.-% auf Trockengewichtsbasis an Magnesiumstearat,
unter Bildung einer zu einer Tablette komprimierbaren DPP-IV-Formulierung in Form eines tablettierbaren Pulvers mischt und
b) die in Schritt a) hergestellte Formulierung unter Bildung der komprimierten DPP-IV-Inhibitortablette in Einheitsdosisform komprimiert.

## Revendications

1. Comprimé pharmaceutique à compression directe, dans lequel le comprimé comprend :
a) de 22% à 28% en poids, sur une base de poids sec, d'un inhibiteur de DPP-IV qui est la (S)-1-[(3-hydroxy-1-adamantyl)amino]acétyl-2-cyanopyrrolidine sous forme libre ou sous forme de sel d'addition d'acide ;
b) de 45% à 50% en poids, sur une base de poids sec, d'une cellulose microcristalline pharmaceutiquement acceptable ;
c) de 20% à 25% en poids, sur une base de poids sec, d'un lactose pharmaceutiquement acceptable ;
d) de 1,5% à 2,5% en poids, sur une base de poids sec, d'un glycolate d'amidon sodique pharmaceutiquement acceptable ; et
e) de 0,1% à 2% en poids, sur une base de poids sec, d'un stéarate de magnésium ;
dans lequel la dispersion contient des particules comprenant ledit inhibiteur de DPP-IV qui est la (S)-1-[(3-hydroxy-1-adamantyl)amino]acétyl-2-cyanopyrrolidine sous forme libre ou sous forme de sel d'addition d'acide, et dans lequel au moins 80% de la distribution granulométrique des particules comprenant la (S)-1-[(3-hydroxy-1-adamantyl)amino]acétyl-2-cyanopyrrolidine sous forme libre ou sous forme de sel d'addition d'acide dans le comprimé est comprise entre 10 µm et 250 µm et au moins 35% de la distribution granulométrique des particules comprenant la (S)-1-[(3-hydroxy-1-adamantyl)amino]acétyl-2-cyanopyrrolidine sous forme libre ou sous forme de sel d'addition d'acide dans le comprimé est comprise entre 50 µm et 150 µm, tel que déterminé par diffraction laser.

2. Comprimé pharmaceutique à compression directe selon la revendication 1, dans lequel le rapport de l'épaisseur du comprimé sur le poids du comprimé est de 0,002 à 0,06 mm/mg.

3. Comprimé pharmaceutique à compression directe selon l'une quelconque des revendications 1 à 2, dans lequel le rapport de l'épaisseur du comprimé sur le poids du comprimé est de 0,01 à 0,03 mm/mg.

4. Comprimé pharmaceutique à compression directe selon l'une quelconque des revendications 1 à 3, dans lequel au moins 45% de la distribution granulométrique des particules comprenant la (S)-1-[(3-hydroxy-1-adamantyl)amino]acétyl-2-cyanopyrrolidine sous forme libre ou sous forme de sel d'addition d'acide dans le comprimé est comprise entre 50 µm et 150 µm, tel que déterminé par diffraction laser.

5. Comprimé pharmaceutique à compression directe selon l'une quelconque des revendications 1 à 4, dans lequel la teneur en eau du comprimé est inférieure à 5% après 1 semaine à 25°C et 60% d'HR.

6. Comprimé pharmaceutique à compression directe selon la revendication 1, dans lequel
i) entre 0 et 10 minutes, 85% à 99,5% de l'ingrédient actif est libéré, et
ii) entre 10 et 15 minutes, 90% à 99,5% de l'ingrédient actif est libéré.

7. Comprimé pharmaceutique à compression directe selon l'une quelconque des revendications 1 à 6, dans lequel la distribution granulométrique des excipients pharmaceutiques dans le comprimé se trouve entre 5 et 400 µm, préférablement entre 10 et 250 µm, tel que déterminé par diffraction laser.

8. Procédé de préparation d'un comprimé compressé selon l'une quelconque des revendications 1 à 7, sous forme de dosage unitaire, qui comprend :
a) le mélange, en tant que pourcentage en poids, sur une base de poids sec :
i) de 22% à 28% en poids, sur une base de poids sec, d'un inhibiteur de DPP-IV qui est la (S)-1-[(3-hydroxy-1-adamantyl)amino]acétyl-2-cyanopyrrolidine sous forme libre ou sous forme de sel d'addition d'acide ;
ii) de 45% à 50% en poids, sur une base de poids sec, d'une cellulose microcristalline pharmaceutiquement acceptable ;
iii) de 20% à 25% en poids, sur une base de poids sec, d'un lactose pharmaceutiquement acceptable ;
iv) de 1,5% à 2,5% en poids, sur une base de poids sec, d'un glycolate d'amidon sodique pharmaceutiquement acceptable ; et
v) de 0,1% à 2% en poids, sur une base de poids sec, d'un stéarate de magnésium ;
afin de former une formulation de DPP-IV sous la forme d'une poudre tablettaire, capable d'être compressée en un comprimé ; et
b) la compression de la formulation préparée lors de l'étape a) afin de former le comprimé d'inhibiteur de DPP-IV compressé, sous forme de dosage unitaire.
